(19) Europäisches Patentamt
European Patent Office
Office européen des brevets

(11) Veröffentlichungsnummer: **0 219 799**
**B1**

(12) # EUROPÄISCHE PATENTSCHRIFT

(45) Veröffentlichungstag der Patentschrift: **14.12.88**

(51) Int. Cl.[4]: **C 07 D 301/04**, C 07 D 405/06

(21) Anmeldenummer: **86114185.1**

(22) Anmeldetag: **14.10.86**

(54) **Verfahren zur Herstellung von Oxiranen.**

(30) Priorität: **24.10.85 DE 3537817**

(43) Veröffentlichungstag der Anmeldung: **29.04.87 Patentblatt 87/18**

(45) Bekanntmachung des Hinweises auf die Patenterteilung: **14.12.88 Patentblatt 88/50**

(84) Benannte Vertragsstaaten: **AT BE CH DE FR GB IT LI NL**

(56) Entgegenhaltungen:
**CHEMICAL ABSTRACTS, Band 104, nr. 9, 3. März 1986, Columbus, Ohio, USA, P. MOSSET; R. GREE "Trimethylsulfonium methyl sulfate, a simple and efficient epoxidizing agent", p. 666, Zusammenfassung nr. 68 688b**
**CHEMICAL ABSTRACTS, Band 102, nr. 17, 29. April 1985, Columbus, Ohio, USA, K. KLOC; E. KUBICZ; J. MLOCHOWSKI "A novel approach to functionalization of azines. Oxiranyl and thiiranyl derivatives of pyridine, quinoline, and isoquinoline", p. 584, Zusammenfassung nr. 149 006p**
**CHEMICAL ABSTRACTS, Band 96, Nr. 13, 29. März 1982, Columbus, Ohio, USA, R.M. MUNAVU; J.A. OGUR; "the preparation of trimethylsulfonium bromide and its utility in the synthesis of oxiranes", p. 684, Zusammenfassung Nr. 103 999u**
**CHEMICAL ABSTRACTS, Band 82, Nr. 5, 3. Februar 1975, Columbus, Ohio, USA, M. NAGAYAMA; O.**

(73) Patentinhaber: **BAYER AG, Konzernverwaltung RP Patentabteilung, D-5090 Leverkusen 1 Bayerwerk (DE)**

(72) Erfinder: **Reiser, Wolf, Dr., Kiebitzweg 12a, D-5600 Wuppertal 1 (DE)**
Erfinder: **Feyen, Peter, Dr., Mozartstrasse 1, D-4020 Mettmann (DE)**

(56) Entgegenhaltungen: (Fortsetzung)
**OKUMURA; K. YAGUCHI; A. MORI "Reaction of alpha-olefins and epoxyalkanes with the dimethyl sulfide-sulfur trioxide complex", p. 465, Zusammenfassung Nr. 30 900d**

**EP 0 219 799 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein neues Verfahren zur Herstellung von bekannten Oxiranen, die als Zwischenprodukte zur Synthese von Verbindungen mit pflanzenwuchsregulierender und fungizider Wirksamkeit verwendet werden können.

Es ist bereits bekannt geworden, daß man Oxirane herstellen kann, indem man Dimethylsulfid mit Dimethylsulfat umsetzt und das dabei intermediär entstehende Trimethylsulfonium-methylsulfat anschließend mit Carbonyl-Verbindungen in Gegenwart eines inerten organischen Lösungsmittels und in Gegenwart einer starken Base, wie Butyllithium, Natriumhydrid, Natriumamid, Kalium-tert.butylat, Natriummethylat oder Natriumethylat zur Reaktion bringt (vgl. J. Amer. Chem. Soc. 87, 1363 - 1364 (1965) und Ber. 96, 1881 (1963)). So läßt sich zum Beispiel 2-(4-Chlorophenoxy-methyl)-2-tert.-butyloxiran herstellen, indem man aus Dimethylsulfid und Dimethylsulfat zubereitetes Trimethylsulfonium-methylsulfat in situ mit 1-(4-Chlorphenoxyl-3-3-dimethyl-butan-2-on in Acetonitril in Gegenwart von Natriummethylat umsetzt (vgl. EP-OS-40 345). Die Ausbeuten bei diesem Verfahren sind gut. Nachteilig ist jedoch, daß die verwendbaren Basen jeweils speziell hergestellt werden müssen und schwierig zu handhaben sind, da sie feuchtigkeitsempfindlich und zum Teil auch brennbar sind. Beeinträchtigend ist auch, daß sowohl bei der Herstellung des Trimethylsulfoniummethylsulfates als auch bei dessen anschließender Umsetzung mit dem Keton relativ lange Reaktionszeiten erforderlich sind.

Weiterhin ist bekannt, daß man Oxirane erhält, wenn man Dimethylsulfid mit Dimethylsulfat behandelt und das dabei anfallende Trimethylsulfoniummethylsulfat mit Carbonyl-Verbindungen in Gegenwart von konzentrierter wäßriger Natronlauge, einem mit Wasser wenig mischbaren organischen Lösungsmittel sowie in Gegenwart eines Phasen-Transfer-Katalysators umsetzt (Vgl. Angew. Chem 85, 867 - 868 (1973) und J. Org. Chem. 34, 2133 (1969)). Nach diesem Verfahren wurden bisher allerdings nur Aldehyde in Oxirane überführt. Außerdem ist in dem aus zwei flüssigen Phasen bestehenden System jeweils die Anwesenheit eines Phasen-Transfer-Katalysators erforderlich.

Schließlich ist bereits bekannt, daß sich Oxirane synthetisieren lassen, indem man Dimethylsulfid mit Dimethylsulfat in Gegenwart eines inerten organischen Verdünnungsmittels, wie z. B. Acetonitril, behandelt und das dabei entstehende Trimethylsulfonium-methylsulfat ohne vorherige Isolierung mit bestimmten Ketonen in Gegenwart von festem Kaliumhydroxid oder Natriumhydroxid und in Gegenwart eines inerten organischen Verdünnungsmittels, wie z. B. Toluol, umsetzt (vgl. DE-OS-3 315 619). Die Ausbeuten an den gewünschten Produkten sind sehr gut. Ein entscheidender Nachteil dieses Verfahrens besteht allerdings darin, daß sowohl bei der Zubereitung des Trimethylsulfonium-methylsulfats als auch bei dessen Umsetzung mit Keton jeweils realtiv lange Reaktionszeiten benötigt werden.

Es wurde nun gefunden, daß man die bekannten Oxirane der Formel

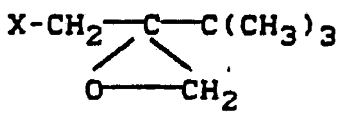 (I)

in welcher

X für 4-Chlorbenzyl, 4-Phenylphenoxy oder 1H-(1,2,4)-Triazolyl steht,

erhält, wenn man Dimethylsulfat mit einem Überschuß an Dimethylsulfid behandelt und das dabei entstehende Trimethylsulfonium-methylsulfat der Formel

$$(CH_3)_3S^{\oplus} \quad CH_3SO_4^{\ominus} \qquad (II)$$

ohne vorherige Isolierung mit einem Keton der Formel

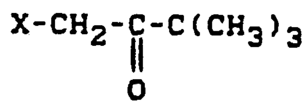 (III)

in welcher

X die oben angegebene Bedeutung hat,

in Gegenwart von festem Kaliumhydroxid oder Natriumhydroxid in Dimethylsulfid, gegebenenfalls in Gegenwart eines polaren, aprotischen Verdünnungsmittels, bei Temperaturen zwischen 0°C und 50°C umsetzt.

Es ist als äußerst überraschend zu bezeichnen, daß sich Oxirane der Formel (I) nach dem erfindungsgemäßen Verfahren in wesentlich kürzeren Reaktionszeiten als bei den aus der EP-OS-0 040 345 und der DE-OS-3 315 619 bekannten Verfahren in extrem hoher Ausbeute herstellen lassen.

Das erfindungsgemäße Verfahren zeichnet sich durch eine Reihe von Vorteilen aus. So sind die einsetzbaren Basen auch in technischem Maßstab verfügbar, einfach zu handhaben und nicht brennbar. Ferner kann das im Überschuß vorhandene Dimethylsulfid, das sowohl als Reaktionskomponente als auch als Verdünnungsmittel

fungiert, ohne Schwierigkeiten zurückgewonnen und bei weiteren Umsetzungen erneut eingesetzt werden. Da es sich hierbei um ein niedrig siedendes Verdünnungsmittel handelt, sind die zur Erwärmung auf die Reaktionstemperatur und zur Recyclisierung erforderlichen Energiemengen realtiv gering.

Von Vorteil ist auch die einfache Aufarbeitung des nach der Umsetzung anfallenden Reaktionsgemisches. Gegebenenfalls im Reaktionsgemisch enthaltenes polares aprotisches Verdünnungsmittel geht bei der Aufarbeitung vollständig in die wäßrige Phase über. Zu erwähnen ist außerdem, daß die Oxirane der Formel (I) nach dem erfindungsgemäßen Verfahren in sehr hoher Ausbeute zugänglich sind.

Verwendet man bei dem erfindungsgemäßen Verfahren 1-(4-Chlorphenyl)-4,4-dimethylpentan-3-on als Ausgangsstoff und festes Kaliumhydroxid als Base, so kann der Reaktionsablauf durch das folgende Formelschema Veranschaulicht werden:

a) $$(CH_3)_2S + (CH_3)_2SO_4 \xrightarrow[(CH_3)_2S]{} (CH_3)_3S^{\ominus}\quad CH_3SO_4^{\ominus}$$

b) 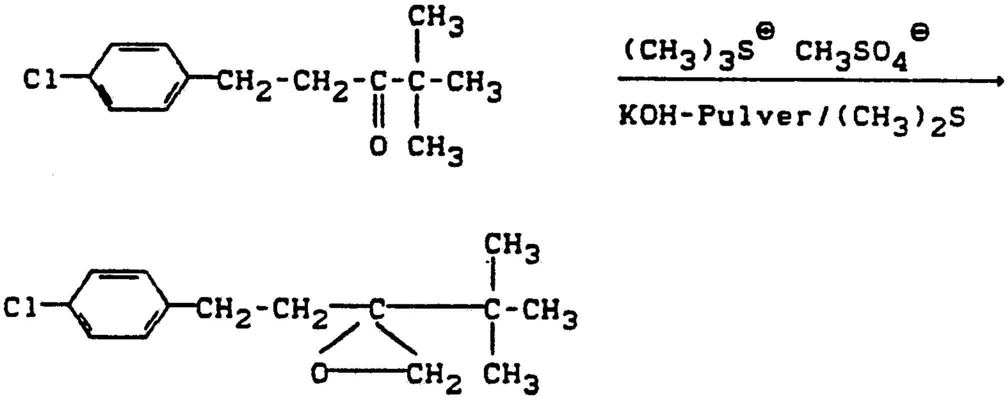


Die bei dem erfindungsgemäßen Verfahren als Ausgangsstoffe benötigten Ketone sind durch die Formel (III) definiert. In dieser Formel steht X für 4-Chlorbenzyl, 4-Phenylphenoxy oder 1H-(1,2,4)-Triazolyl.

Als Beispiele für Ketone der Formel (III) seien diejenigen Stoffe erwähnt, die nachstehend formelmäßig aufgeführt sind:

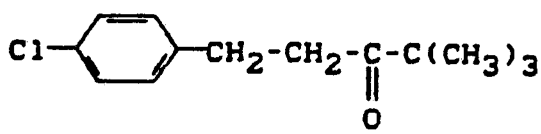

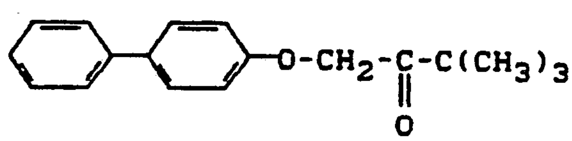 und

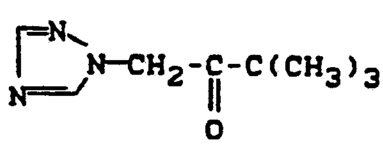

Die Ketone der Formel (III) sind bereits bekannt (vgl. DE-OS-3 315 619, DE-OS-3 315 681 und DE-OS-3 111 238).

Das bei dem erfindungsgemäßen Verfahren weiterhin als Ausgangsstoff benötigte Trimethylsulfonium-methylsulfat der Formel (II) ist ebenfalls bekannt (vgl. Heterocycles 8, 397 (1977)). Es wird bei der obigen Umsetzung in frisch hergestelltem Zustand eingesetzt, indem man es durch Reaktion von Dimethylsulfid mit

Dimethylsulfat in situ erzeugt.

Als Basen dienen bei dem erfindungsgemäßen Verfahren Kaliumhydroxid oder Natriumhydroxid in fester Form. Sie können als Pellets oder Pulver eingesetzt werden.

Als Verdünnungsmittel fungiert bei dem erfindungsgemäßen Verfahren Dimethylsulfid, und zwar sowohl bei der Herstellung des Trimethylsulfonium-methylsulfats als auch bei dessen anschließender Umsetzung mit einem Keton der Formel (III). Es ist jedoch auch möglich, Dimethylsulfid im Gemisch mit einem polaren, aprotischen Solvens, wie z. B. N-Methylpyrrolidon, Dimethylformamid oder Dimethylsulfoxid, als Verdünnungsmittel zu verwenden.

Die Reaktionstemperaturen können bei der Durchführung des erfindungsgemäßen Verfahrens innerhalb eines bestimmten Bereiches variiert werden. Im allgemeinen arbeitet man sowohl bei der Herstellung des Trimethylsulfonium-methylsulfats als auch bei dessen anschließender Umsetzung mit einem Keton der Formel (III) bei Temperaturen zwischen 0°C und 50°C. Vorzugsweise arbeitet man bei der Herstellung des Trimethylsulfonium-methylsulfats bei Temperaturen zwischen 20 und 40°C, und bei der nachfolgenden Umsetzung des Salzes mit einem Keton der Formel (III) bei Temperaturen zwischen 15°C und 40°C, was der Rückflußtemperatur des Verdünnungsmittels entspricht.

Das erfindungsgemäße Verfahren wird bei Normaldruck durchgeführt.

Die Reaktionszeiten liegen bei dem erfindungsgemäßen Verfahren im allgemeinen zwischen 1 und 16 Stunden, vorzugsweise zwischen 1,5 und 8 Stunden.

Bei der Durchführung des erfindungsgemäßen Verfahrens werden die Mengen an Reaktionskomponenten und Verdünnungsmittel so gewählt, daß man auf 1 Mol an Keton der Formel (III) im allgemeinen 2 bis 15 Mol, vorzugsweise 3 bis 10 Mol an Dimethylsulfid, und im allgemeinen 1 bis 3 Mol, vorzugsweise 1,2 bis 2,0 Mol an Dimethylsulfat sowie im allgemeinen 1 bis 6 Mol, vorzugsweise 1,5 bis 4 Mol an Kaliumhydroxid bzw. Natriumhydroxid einsetzt.

Im einzelnen verfährt man bei der Umsetzung nach dem erfindungsgemäßen Verfahren in der Weise, daß man Dimethylsulfat mit einem Überschuß an Dimethylsulfid zusammengibt, dieses Gemisch dann mit einer Lösung von Keton der Formel (III) in Dimethylsulfid, gegebenenfalls im Gemisch mit einem polaren, aprotischen Solvens, versetzt und anschließend die jeweils gewünschte Menge an Base hinzufügt. Die Aufarbeitung erfolgt nach üblichen Methoden. Im allgemeinen geht man so vor, daß man das Reaktionsgemisch mit Wasser versetzt, die Phasen trennt, die organische Phase durch Zugabe von verdünnter Mineralsäure neutralisiert, die Phasen trennt, die organische Phase mit Wasser wäscht und durch Abdestillieren des Verdünnungsmittels bei Normaldruck einengt. Das dabei anfallende Produkt kann zurweiteren Reinigung unter vermindertem Druck destilliert werden.

Die nach dem erfindungsgemäßen Verfahren herstellbaren Oxirane der Formel (I) sind wertvolle Ausgangsstoffe zur Synthese von 1-Hydroxyethyl-azol-Derivaten, welche hervorragende pflanzenwuchsregulierende und fungizide Eigenschaften besitzen (vgl. EP-OS-0 040 345 und DE-OS-3 237 400).

So läßt sich beispielsweise das 1-(4-Chlorphenyl)-3-(1,2,4-triazol-1-yl-methyl)-4,4-dimethyl-pentan-3-ol herstellen, indem man 2-(4-Chlorphenyl-ethyl)-2-tert.-butyl-oxiran mit 1,2,4-Triazol in Gegenwart von Natriumhydroxid umsetzt. Diese Synthese läßt sich durch das folgende Formelschema wiedergeben:

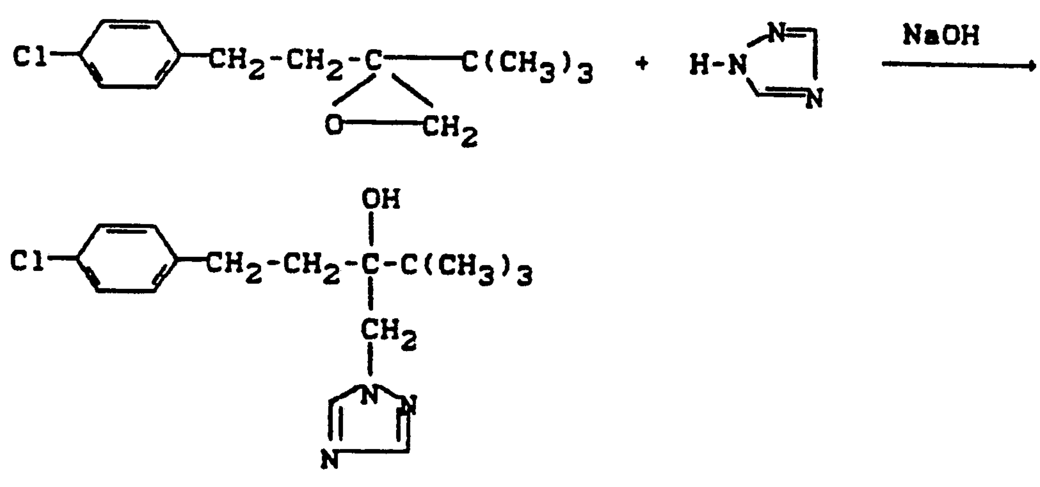


Ferner läßt sich das 3,3-Dimethyl-2-(4-methoximinomethyl-phenoxymethyl)-1-(1,2,4-triazol-1-yl)-butan-2-ol herstellen, indem man 2-(1,2,4-Triazol-1-yl-methyl)-2-tert.-butyl-oxiran mit 4-Methoximinomethyl-phenol in Gegenwart von Kaliumcarbonat umsetzt. Diese Synthese läßt sich durch das folgende Formelschema wiedergeben:

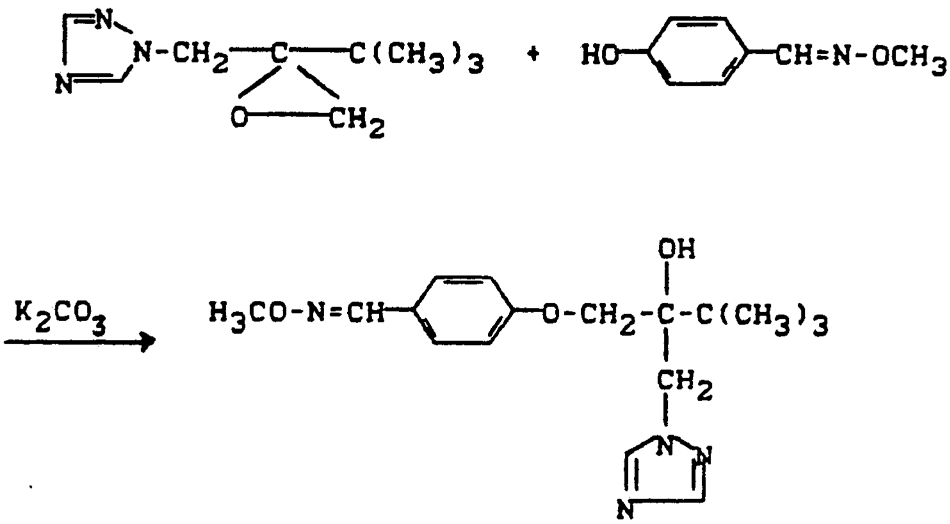


Das erfindungsgemäße Verfahren und die Herstellung von Endprodukten aus den Oxiranen der Formel (I) werden durch die folgenden Beispiele veranschaulicht.

**Beispiel 1**

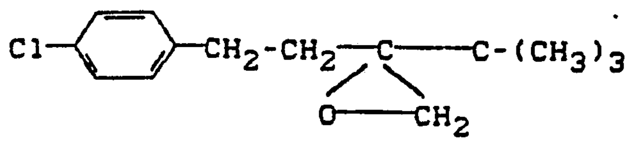

In ein Gemisch aus 744 g (12 Mol) Dimethylsulfid, 72,5 ml Dimethylsulfoxid und 453,2 g (2 Mol) 1-(4-Chlorphenyl)-4,4-dimethyl-pentan-3-on wurden 378,4 g (3 Mol) Dimethylsulfat unter Rühren bei 33°C so zugetropft, daß sich das Reaktionsgemisch auf 40°C erwärmte. Nach beendeter Zugabe ließ man 2 Stunden bei 40°C nachrühren, kühlte dann das Gemisch auf 18°C ab und gab 448 g (7 Mol) Kaliumhydroxid-Pulver portionsweise hinzu, wobei sich das Reaktionsgemisch auf 27°C erwärmte. Anschließend wurde nach 3 Stunden bei 40°C gerührt. Gemäß Gaschromatogramm wiesen die aus dem Reaktionsgemisch gezogenen Proben eine Stunde nach der Kaliumhydroxid-Zugabe einen Gehalt von 70,5 %, nach 2 Stunden 89,8 % und nach 3 Stunden einen Gehalt von 94,3 % an 2-(4-Chlorphenylethyl)-2-tert.-butyl-oxiran auf.

**Beispiel 2**

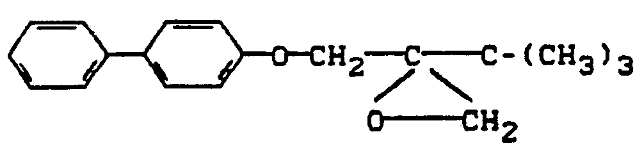

Zu 186 g (3 Mol) Dimethylsulfid wurden bei 40°C unter Rühren innerhalb von 20 Minuten 75,6 g (0,6 Mol) Dimethylsulfat getropft. Danach wurde das Gemisch noch 2 Stunden bei Raumtemperatur gerührt. Nach anschließender Zugabe von 11,3 ml Dimethylsulfoxid und 134 g (0,5 Mol) 1-(4-Phenylphenoxy)-3,3-dimethyl-butan-2-on wurden 56 g (0,9 Mol) Kaliumhydroxid-Pulver portionsweise hinzugefügt. Die aus dem Reaktionsgemisch nach der Kaliumhydroxid-Zugabe entnommenen Proben wiesen gemäß Gaschromatogramm nach 1 Stunde 86,6 %, und nach 2 Stunden 95,9 % an 1-(4-Phenyl-phenoxy-methyl)-2-tert.-butyl-oxiran auf.

Anschließend wurde das Gemisch mit 200 ml Wasser versetzt. Die organische Phase wurde abgetrennt und mit verdünnter wäßriger Salzsäure neutralisiert. Erneut wurde die organische Phase abgetrennt, dann mit Wasser gewaschen und durch Destillation bei Normaldruck eingeengt. Man erhielt 128,3 g eines Rückstandes, der gemäß Gaschromatogramm zu 92,8 % aus 1-(4-Phenyl-phenoxymethyl)-2-tert.-butyl-oxiran bestand.

**Beispiel 3**

25 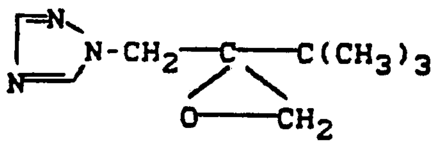

163,8 g (1,3 Mol) Dimethylsulfat wurden bei einer Temperatur von 36°C unter Rühren innerhalb von 2 Stunden in 592 g (9,55 Mol) Dimethylsulfid eingetropft. Nach vierstündigem Rühren bei 36°C wurde das Reaktionsgemisch auf Raumtemperatur abgekühlt und nacheinander mit 167 g (1 Mol) 1-C1H-1,2,4-Triazolyl)-3-3-dimethyl-butan-2-on und 95,45 g (1,5 Mol) Kaliumhydroxidpulver versetzt. Die aus dem Reaktionsgemisch nach der Kaliumhydroxid-Zugabe entnommenen Proben wiesen gemäß Gaschromatogramm nach 0,5 Stunden 73,9 % und nach 3 Stunden 79,5 % an 2-[(1H)-(1,2,4-Triazolyl-methyl]-2-tert.-butyl-oxiran auf.

**Vergleichsbeispiel**

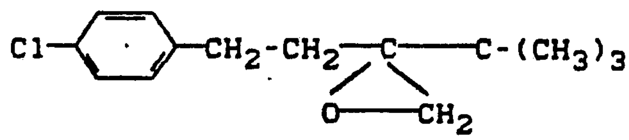

In ein Gemisch aus 88 ml Toluol, 22 ml Dimethylsulfoxid und 38 g (0,3 Mol) Dimethylsulfat wurden unter Rühren 18,9 g (0,3 Mol) Dimethylsulfid eingetropft, wobei die Temperatur des Gemisches auf 52°C anstieg. Das Gemisch wurde noch 4 Stunden bei Raumtemperatur nachgerührt und dann mit 46,8 g (0,2 Mol) 1-(4-Chlorphenyl)-4-4-dimethyl-pentan-3-on versetzt. Anschließend wurden 44,8 g (0,7 mol) Kaliumhydroxid-Pulver hinzugefügt, wobei die Temperatur des Gemisches durch Kühlung auf 40°C gehalten wurde. Danach wurde auf Raumtemperatur abgekühlt. Die aus dem Reaktionsgemisch nach der Kaliumhydroxid-Zugabe entnommenen Proben wiesen gemäß Gaschromatogramm nach 1,5 Stunden 57,5 %, nach 3 Stunden 65,0 % und nach 20 Stunden 92,1 % an 2-(4-Chlorphenyl-ethyl)-2-tert.-butyl-oxiran auf.

Beispiele für die Verwendung erfindungsgemäß hergestellter Oxirane zur Synthese von 1-Hydroxyethyl-azol-Derivaten mit fungizider und pflanzenwuchsregulierender Wirksamkeit:

**Beispiel A**

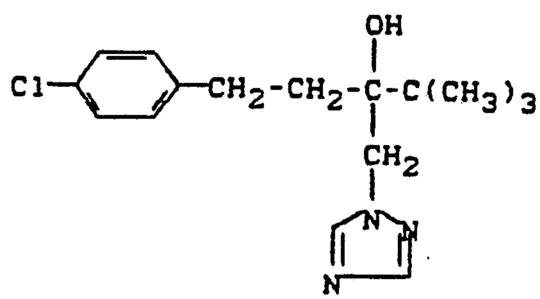

Ein Gemisch aus 245,2 g (1 Mol) 2-(4-Chlorphenyl-ethyl)-2-tert.-butyl-oxiran (97,2 %-ig), 72,5 g (1 Mol) 1,2,4-Triazol (95,2 %-ig) und 380 ml Dimethylsulfoxid wurde bei Raumtemperatur unter Rühren mit 5,3 g (0,05 Mol) Natriumcarbonat versetzt und dann 4 Stunden auf 110°C erhitzt. Das Reaktionsgemisch wurde danach auf 1 Liter Wasser gegossen und mit verdünnter wäßriger Salzsäure neutralisiert. Die flüssige Phase wurde vom Feststoff abdekantiert, und der Rückstand wurde in 1 Liter Toluol gelöst. Man extrahierte mit Wasser, trennte die Phasen, trocknete die organische Phase und engte durch Abziehen des Verdünnungsmittels unter vermindertem Druck ein. Man erhielt auf diese Weise 128 g (42 % der Theorie) an 1-(4-Chlorphenyl)-3-(1,2,4-triazol-1-yl-methyl)-4,4-dimethyl-pentan-3-ol in Form eines Feststoffes.

**Beispiel B**

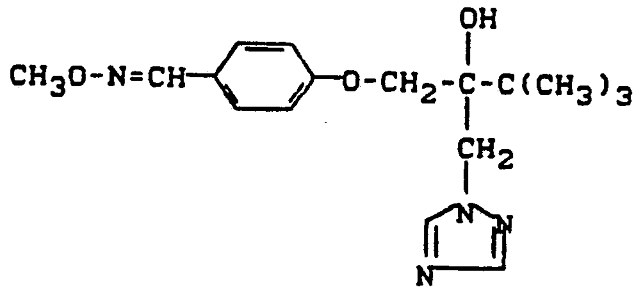

35

Ein Gemisch aus 151,0 g (1 Mol) 4-Methoximinomethyl-phenol (100 %-ig), 222,3 g (1 Mol) 2-(1,2,4-Triazol-1-yl-methyl)-2-tert.-butyl-oxiran (81,7 %-ig) und 13,8 g (0,1 Mol) gemahlenem Kaliumcarbonat in 500 ml Dimethylsulfoxid wurde 24 Stunden auf 110°C erhitzt und anschließend unter vermindertem Druck eingeengt. Man versetzte den Rückstand mit 500 ml Isopropanol, tropfte 600 ml Wasser hinzu und kühlte das Gemisch auf 5°C ab. Nach Zugabe von Impfkristallen wurde 16 Stunden bei Raumtemperatur gerührt, und danach wurde der ausgefallene Niederschlag abfiltriert. Dieser Rückstand wurde mit 250 ml 50 %-igem wäßrigen Isopropanol gewaschen und anschließend getrocknet. Man erhielt auf diese Weise 270,3 g eines Produktes, das gemäß Gaschromatogramm zu 80,1 %. aus 3,3-Dimethyl-2-(4-methoximino-methyl-phenoxymethyl)-1-(1,2,4-triazol-1-yl)-butan-2-ol bestand. Die Ausbeute errechnet sich danach zu 65,1 % der Theorie.

**Patentansprüche**

1. Verfahren zur Herstellung von Oxiranen der Formel

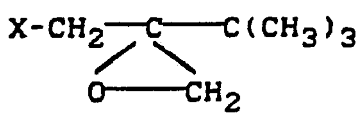

(I)

in welcher
X für 4-Chlorbenzyl, 4-Phenylphenoxy oder 1H-(1,2,4)-Triazolyl steht,
dadurch gekennzeichnet, daß man Dimethylsulfat mit einem Überschuß an Dimethylsulfid behandelt und das dabei entstehende Trimethylsulfonium-methylsulfat der Formel

$$(CH_3)_3S^{\oplus} \quad CH_3SO_4^{\ominus} \qquad (II)$$

ohne vorherige Isolierung mit einem Keton der Formel

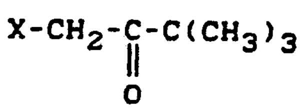

in welcher
X die oben angegebene Bedeutung hat,
in Gegenwart von festem Kaliumhydroxid oder Natriumhydroxid in Dimethylsulfid, gegebenenfalls in Gegenwart eines polaren, aprotischen Verdünnungsmittels, bei Temperaturen zwischen 0°C und 50°C umsetzt.

2. Verfahren gemäß Anspruch 1, dadurch gekennzeichnet, daß man bei der Behandlung von Dimethylsulfat mit Dimethylsulfid bei Siedetemperatur des Dimethylsulfids arbeitet.

3. Verfahren gemäß Anspruch 1, dadurch gekennzeichnet, daß man bei der Umsetzung des Trimethylsulfoniummethylsulfats der Formel (II) mit einem Keton der Formel (III) bei Temperaturen zwischen 20° C und 40°C arbeitet.

4. Verfahren gemäß Anspruch 1, dadurch gekennzeichnet, daß man auf 1 Mol an Keton der Formel (III) 2 bis 15 Mol an Dimethylsulfid, 1 bis 3 Mol an Dimethylsulfat und 1 bis 6 Mol an Kaliumhydroxid oder Natriumhydroxid einsetzt.

5. Verfahren gemäß Anspruch 1, dadurch gekennzeichnet, daß man 1-(4-Chlorphenyl)-4,4-dimethyl-pentan-3-on als Ausgangsstoff der Formel (III) einsetzt.

## Claims

1. Process for the preparation of oxiranes of the formula

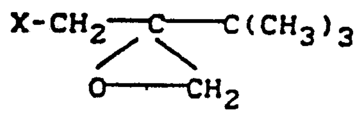

(I)

in which

X represents 4-chlorobenzyl, 4-phenylphenoxy or 1H-(1,2,4)-triazolyl,

characterized in that dimethyl sulphate is treated with an excess of dimethyl sulphide and the trimethylsulphonium methylsulphate thereby formed, of the formula

$$(CH_3)_3S^{\oplus} \; CH_3SO_4^{\ominus} \qquad (II),$$

is reacted, without prior isolation, with a ketone of the formula

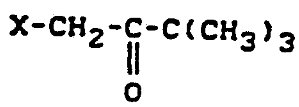

in which

X has the abovementioned meaning

in the presence of solid potassium hydroxide or sodium hydroxide in dimethyl sulphide, optionally in the presence of a polar, aprotic diluent, at temperatures between 0° C and 50° C.

2. Process according to Claim 1, characterized in that the treatment of dimethyl sulphate with dimethyl sulphide is carried out at the boiling point of the dimethyl sulphide.

3. Process according to Claim 1, characterized in that the reaction of the trimethylsulphonium methylsulphate of the formula (II) with a ketone of the formula (III) is carried out at temperatures between 20° C and 40° C.

4. Process according to Claim 1, characterized in that 2 to 15 moles of dimethyl sulphide, 1 to 3 moles of dimethyl sulphate and 1 to 6 moles of potassium hydroxide or sodium hydroxide are employed per mole of ketone of the formula (III).

5. Process according to Claim 1, characterized in that 1-(4-chlorophenyl)-4,4-dimethyl-pentan-3-one is employed as the starting material of the formula (III).

## Revendications

1. Procédé pour la production d'oxiranes répondant à la formule

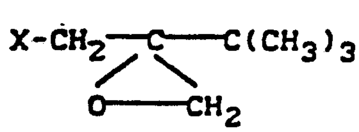

(I)

dans laquelle

X représente du 4-chlorobenzyle, du 4-phénylphénoxy ou du 1H-(1,2,4)-triazolyle,

caractérisé en ce que l'on traite le sulfate de diméthyle par un excès de sulfure de diméthyle et que le sulfate de triméthylsulfonium -méthyle ainsi obtenu répondant à la formule

$$(CH_3)_3S^{\oplus} \quad CH_3SO_4^{\ominus} \qquad (II)$$

est mis à réagir sans séparation préalable avec une cétone répondant à la formule

$$X-CH_2-\underset{\underset{O}{\|}}{C}-C(CH_3)_3 \qquad (III)$$

dans laquelle

X a la signification précitée
en présence d'hydroxyde de potassium, ou d'hydroxyde de sodium solide, dans le sulfure de diméthyle, éventuellement en présence d'un diluant aprotique polaire, à des températures comprises entre 0° C et 50° C.

2. Procédé selon la revendication 1, caractérisé en ce que l'on opère à la température d'ébullition du sulfure de diméthyle pour traiter le sulfate de diméthyle par le sulfure de diméthyle.

3. Procédé selon la revendication 1, caractérisé en ce que l'on opère à des températures comprises entre 20°C et 40°C pour réaliser la réaction du sulfate de triméthylsulfonium-méthyle de la formule (II) avec une cétone répondant à la formule (III).

4. Procédé selon la revendication 1, caractérisé en ce que l'on utilise pour une mole de cétone répondant à la formule (III) de 2 à 15 moles de sulfure de diméthyle, de 1 à 3 moles de sulfate de diméthyle et de 1 à 6 moles d'hydroxyde de potassium ou d'hydroxyde de sodium.

5. Procédé selon la revendication 1, caractérisé en ce que l'on utilise comme matière première répondant à la formule (III) la 1-(4-chlorophényl)-4,4-diméthyl-pentane-3-one.